# FASCICULE DE BREVET EUROPEEN

(11) **EP 3 554 423 B1**
(45) Date de publication et mention de la délivrance du brevet: **05.08.2020**
(21) Numéro de dépôt: 17822385.5
(22) Date de dépôt: 07.12.2017
(51) Int. Cl.: A61F 2/24

(54) **DISPOSITIF POUR RÉALISER OU PRÉPARER UNE ANNULOPLASTIE MITRALE PAR VOIE TRANSFEMORALE**
VORRICHTUNG ZUR DURCHFÜHRUNG ODER VORBEREITUNG EINER MITRALKLAPPENANULOPLASTIK DURCH EINEN TRANSFEMORALEN ANSATZ
DEVICE FOR PERFORMING OR PREPARING FOR A MITRAL VALVE ANNULOPLASTY BY A TRANSFEMORAL APPROACH

(30) Priorité: 15.12.2016 FR 1662559
(43) Date de publication de la demande: 23.10.2019
(73) Titulaire: CMI'NOV, 43120 Monistrol-sur-Loire (FR); Vola, Marco, 42270 Saint-Priest-en Jarez (FR)
(72) Inventeur: VOLA, Marco, 42270 Saint-Priest en Jarez (FR); PAIN, Bernard, 43120 Monistrol sur Loire (FR)
(74) Mandataire: Cabinet Laurent & Charras
(86) Numéro de dépôt international: PCT/FR2017/053444
(87) Numéro de publication internationale: WO 2018/109329

(56) Documents cités:
- WO-A2-2007/136783
- WO-A2-2014/195786
- US-A1- 2011 257 728
- US-A1- 2013 331 930

## Description

### DOMAINE TECHNIQUE

L'invention concerne un dispositif pour réaliser ou préparer une annuloplastie par voie transfémorale de la valve mitrale d'un cœur.

Autrement dit, l'annuloplastie a pour but de diminuer le calibre de l'anneau mitral en raccourcissant, par plicature de l'attache de la petite valve, le point d'appui étant pris entre les commissures. Par commissure, on entend rétrécissement du périmètre de la partie postérieure de l'anneau mitral en effectuant sur celui-ci des plicatures par des points, avec comme résultat, une diminution du diamètre antéro-postérieur et latéro-latéral de la valve mitrale.

L'invention trouve également une application avantageuse pour la mise en place, sur l'anneau mitral, d'un anneau préventif, dit de préparation ou d'accroche, destiné à recevoir ultérieurement un implant de valve mitrale.

### ETAT ANTERIEUR DE LA TECHNIQUE

Une annuloplastie mitrale est effectuée en tant que moyen de correction d'une fuite mitrale dont le mécanisme est une dilatation de l'anneau mitral (avec perte de coaptation des berges valvulaires) ou, en complément de la correction de fuite avec un autre mécanisme, (prolapsus de la valve mitrale) pour augmenter la coaptation de la valve mitrale postérieure par rapport à la valve mitrale antérieure

Une annuloplastie mitrale constitue une opération longue et lourde qui nécessite l'ouverture d'une des cavités cardiaques cœur et de la cage thoracique avec circulation sanguine extracorporelle.

Une solution connue de l'état de la technique est d'utiliser la voie transapicale, c'est-à-dire de passer directement au niveau de l'apex du cœur. Une solution de ce type ressort par exemple de l'enseignement du document WO 2014/147336.

Ce document décrit un dispositif destiné à être positionné dans un introducteur étanche disposé dans la cavité thoracique entre deux côtes pour pénétrer dans le ventricule gauche en passant par l'apex du cœur. Le dispositif comprend un corps équipé d'une poignée et d'au moins un organe de commande apte à agir sur un ensemble pour la mise en place et la fixation d'une tresse au niveau de l'anneau mitral au moyen d'éléments de suture. Ce dispositif est avantageux en ce que l'ensemble présente des moyens aptes à permettre l'extraction de la suture au travers de l'anneau mitral en étant apte à enserrer la tresse et s'ancrer sur la périphérie de l'anneau mitral sous un effet de pincement de ladite suture en exerçant deux forces d'appui de pression opposées.

La fixation de l'implant par rapport à l'anneau mitral est améliorée en exerçant une force d'appui et une force de contre-appui opposées pour effectuer une perforation avec précision et un passage d'un matériel d'ancrage dans un temps relativement court et sans fragiliser le tissu adjacent.

Cependant, ce type de dispositif peut encore être amélioré. En effet, le dispositif de l'état de la technique est conçu pour passer par la cavité thoracique entre deux côtes pour pénétrer dans le ventricule gauche en passant par l'apex du cœur, et nécessite ainsi une intervention chirurgicale lourde et invasive.
Le document WO2014/195786 décrit un art antérieur conforme au préambule de la revendication 1.

### EXPOSE DE L'INVENTION

L'invention s'est fixée pour but de remédier à aux inconvénients de l'état de la technique d'une manière sûre, simple, efficace et rationnelle.

Le problème que se propose de résoudre l'invention est de faciliter l'opération de l'annuloplastie mitrale.

A cet effet, il a été mis au point un dispositif pour réaliser ou préparer une annuloplastie par voie transfémorale de la valve mitrale, et destiné à être positionné dans un introducteur étanche disposé dans une veine fémorale pour pénétrer dans l'oreillette gauche du cœur en passant par sa paroi septale.

Selon l'invention, le dispositif comprend un ensemble apte à coopérer avec une poignée assujettie à des moyens de commande pour l'actionnement de l'ensemble pour la mise en place et la fixation d'un anneau de renfort, par exemple en textile, sur l'anneau mitral. L'ensemble est agencé à l'extrémité d'une tige de manipulation et comprend :
- un organe d'appui comprenant une pluralité de bras reliés de manière pivotante à l'extrémité de la tige de sorte à passer, sous l'action des moyens de commande, d'une position repliée le long de la tige à une position déployée écartée de la tige pour prendre appui sous l'anneau mitral de manière uniformément répartie le long de la périphérie de la valve mitrale ;
- un organe de contre-appui comprenant une pluralité de bras, à l'extrémité libre desquels est agencé l'anneau de renfort, les bras sont reliés de manière pivotante à un support disposé coaxialement à la tige de sorte à passer, sous l'action des moyens de commande, d'une position repliée le long de la tige à une position déployée écartée de la tige pour réaliser le contre-appui sur l'anneau mitral et positionner l'anneau de renfort ;
- des moyens aptes à permettre l'extraction de sutures pour fixer l'anneau de renfort sur l'anneau mitral.

De cette manière, et d'une manière avantageuse, le dispositif selon l'invention permet, en combinaison avec une poignée et des moyens d'actionnement qui ne font pas partie de l'invention, de réaliser l'opération d'annuloplastie mitrale par la voie transfémorale en remontant par la veine cave. L'opération est légère et peu invasive. L'ensemble actif du dispositif navigue dans la veine cave où la pression sanguine est relativement faible. Le dispositif permet également de préparer une opération d'annuloplastie ultérieure en permettant la mise en place d'un anneau de renfort sous la forme d'un anneau préventif, dit de préparation ou d'accroche, destiné à recevoir ultérieurement un implant de valve mitrale.

Selon d'autres caractéristiques avantageuses de l'invention, prises isolément ou en combinaison :
- les bras de l'organe d'appui et/ou de l'organe de contre-appui passent simultanément ou sélectivement de la position repliée à la position déployée ;
- les bras de l'organe d'appui et/ou de l'organe de contre-appui sont disposés à l'intérieur d'un fourreau dans la position repliée, et sont poussés hors du fourreau et sont auto-expansibles dans la position déployée ;
- les bras de l'organe d'appui et/ou de l'organe de contre-appui comprennent chacun un marqueur radio-opaque et/ou échographique ;
- les bras de l'organe d'appui et/ou de l'organe de contre-appui sont télescopiques ;
- les moyens aptes à permettre l'extraction d'agrafes comprennent une pluralité de bras reliés de manière pivotante à un support disposé coaxialement à la tige de sorte à passer, sous l'action des moyens de commande, d'une position repliée le long de la tige à une position déployée écartée de la tige en correspondance avec les bras de l'organe de contre-appui, chaque bras comprend intérieurement une agrafes apte à être extraite pour s'ancrer sur la périphérie de la valve mitrale et y fixer l'anneau de renfort ;
- chaque bras de l'organe d'appui est recourbé, articulé ou flexible pour faciliter son passage au travers des cordages de la valve mitrale ;
- l'extrémité de chaque bras de l'organe d'appui peut être constituée de plusieurs branches, aptes à s'écarter les unes des autres après le passage des cordages de la valve mitrale pour former plusieurs points d'appui sous l'anneau mitral ;
- chaque bras de l'organe d'appui comprend intérieurement une agrafe apte à être extraite pour traverser la valve mitrale et fixer l'anneau de renfort ;
- l'extrémité libre de chaque bras de l'organe d'appui comprend une pièce de textile en feutre ou téflon destinée à être plaquée sous la valve mitrale et à être fixée par les agrafes en contre-appui de l'anneau de renfort.

### BREVE DESCRIPTION DES DESSINS

D'autres caractéristiques et avantages de l'invention ressortiront clairement de la description qui en est réalisée ci-après, à titre indicatif et nullement limitatif, en référence aux figures schématiques annexées dans lesquelles :
- la figure 1 illustre un cœur et l'introduction, dans l'oreillette gauche du cœur, d'un l'organe d'appui du dispositif selon une première forme de réalisation de l'invention ;
- la figure 2 illustre en détail de l'organe d'appui de la figure 1, en position repliée ;
- la figure 3 illustre le cœur et le déploiement de l'organe d'appui et son positionnement en appui sous la valve mitrale ;
- la figure 4 illustre en détail l'organe d'appui en position déployée ;
- la figure 5 illustre le cœur et le déploiement d'un organe de contre-appui du dispositif, pour le positionnement d'un anneau de renfort sur l'anneau mitral en contre-appui de l'organe d'appui ;
- la figure 6 illustre l'extraction des aiguilles agencées à l'intérieur des bras de l'organe d'appui, permettant de libérer des sutures pour la fixation de l'anneau de renfort ;
- la figure 7 illustre la libération des sutures par l'intermédiaire des aiguilles de la figure 6;
- la figure 8 illustre le désengagement entre les bras de l'organe de contre-appui et l'anneau de renfort, notamment par coulissement d'un chariot ;
- la figure 9 illustre le retrait de l'organe de contre-appui ;
- la figure 10 illustre le cœur et le retrait de l'organe de contre-appui selon la figure 9 ;
- la figure 11 illustre le cœur et le retrait de l'organe d'appui ;
- la figure 12 illustre le cœur avec l'anneau de renfort fixé sur l'anneau mitral ;
- la figure 13 illustre un cœur et l'introduction, dans l'oreillette gauche du cœur, d'un l'organe d'appui du dispositif selon une deuxième forme de réalisation de l'invention
- la figure 14 illustre en détail de l'organe d'appui de la figure 13, en position repliée ;
- la figure 15 illustre le cœur et le déploiement de l'organe d'appui et son positionnement en appui sous la valve mitrale, avec des pièces de textile en feutre ou téflon disposées à l'extrémité des bras de l'organe d'appui et plaquées sous l'anneau mitral ;
- la figure 16 illustre en détail l'organe d'appui en position déployée ;
- la figure 17 illustre le cœur et le déploiement de organe de contre-appui du dispositif, pour le positionnement d'un anneau de renfort sur l'anneau mitral en contre-appui de l'organe d'appui ;
- la figure 18 illustre en détail l'organe de contre-appui et l'organe d'appui en positions déployées ;
- la figure 19 illustre le cœur et le déploiement des bras que comprennent les moyens d'extractions de sutures, en correspondance avec les bras de l'organe de contre-appui ;
- la figure 20 illustre en détail le déploiement des bras des moyens d'extraction conformément à la figure 19 ;
- la figure 21 illustre le retrait des moyens d'extraction de sutures, après libération desdites sutures et fixation de l'anneau de renfort sur l'anneau mitral ;
- la figure 22 illustre le retrait de l'organe de contre-appui ;
- la figure 23 illustre le retrait de l'organe d'appui ;
- la figure 24 illustre le cœur avec l'anneau de renfort fixé sur l'anneau mitral en contre-appui avec les pièces de textile plaquées de l'autre côté de l'anneau mitral pour sa protection ;
- les figures 25, 26 et 27 illustrent, en perspective, différentes formes de réalisation possibles des sutures de fixation de l'anneau de renfort.

### EXPOSE DETAILLE DE L'INVENTION

Le dispositif (1) selon l'invention permet de réaliser une opération chirurgicale, dite d'annuloplastie mitrale, consistant à réparer l'anneau mitral (2) du cœur (3) d'un patient atteint d'une fuite mitrale. Le dispositif permet également de préparer une opération d'annuloplastie ultérieure en permettant la mise en place d'un anneau de renfort sous la forme d'un anneau préventif, dit de préparation ou d'accroche, destiné à recevoir ultérieurement un implant de valve mitrale.

Le dispositif (1) selon l'invention est destiné à être positionné dans un introducteur étanche de tout type connu et approprié (non représenté) et d'un diamètre inférieur ou égal à 8 mm, disposé dans une veine fémorale pour remonter et pénétrer dans l'oreillette gauche (4) du cœur (3) en passant par sa paroi septale (5).

Le dispositif (1) comprend une tige (6) de manipulation, à l'extrémité de laquelle est agencé un ensemble pour la mise en place et la fixation d'un anneau de renfort (7), par exemple en textile, au niveau de la valve mitrale. L'autre extrémité de la tige (6) est destinée à coopérer avec une poignée assujettie à des moyens de commande, non représentés, par exemple sous la forme d'une gâchette, pour l'actionnement de l'ensemble.

Le dispositif (1) selon l'invention permet, sous contrôle radiographique, après avoir remonté la veine cave, de faire pénétrer l'ensemble dans l'oreillette gauche (4) du cœur (3) en passant au travers de la paroi septale (5). Ensuite, lorsque l'ensemble se trouve dans l'oreillette gauche (4) du cœur (3), il est apte à passer au travers de la valve mitrale pour venir se déployer côté ventricule en dessous de ladite valve mitrale et prendre appui sous l'anneau mitral (2).

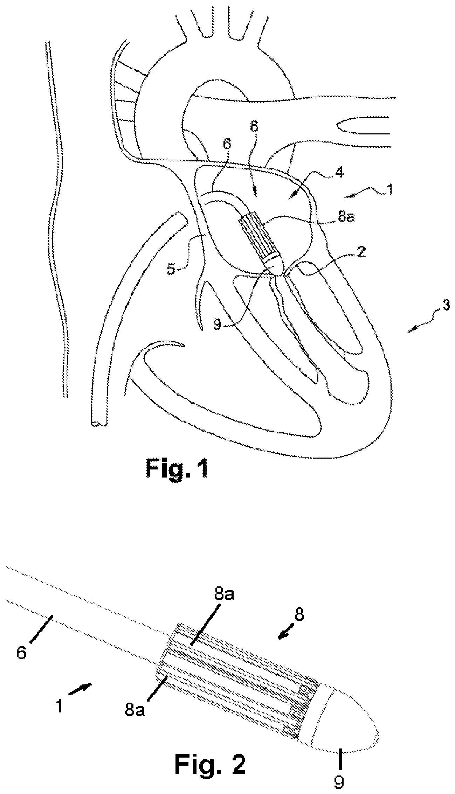

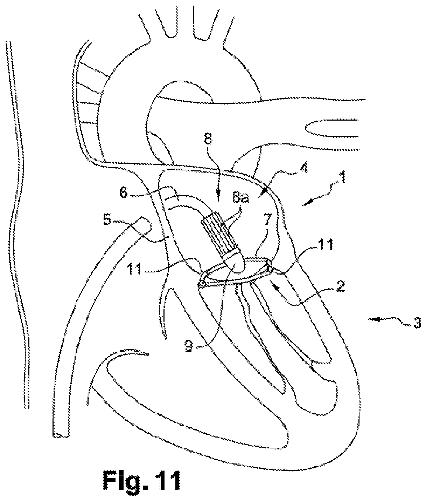

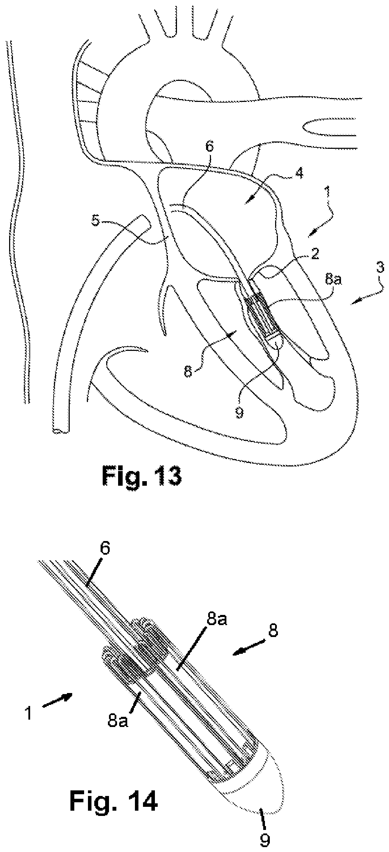

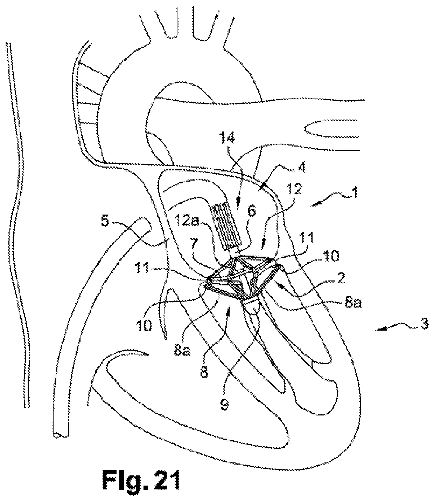

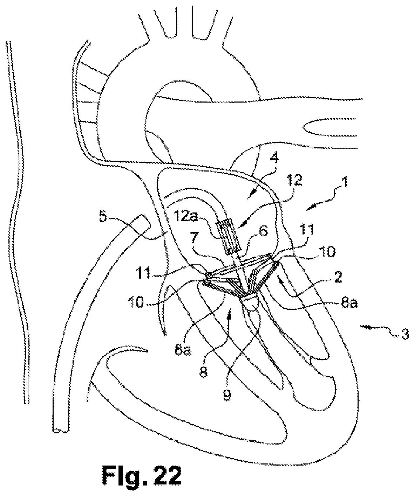

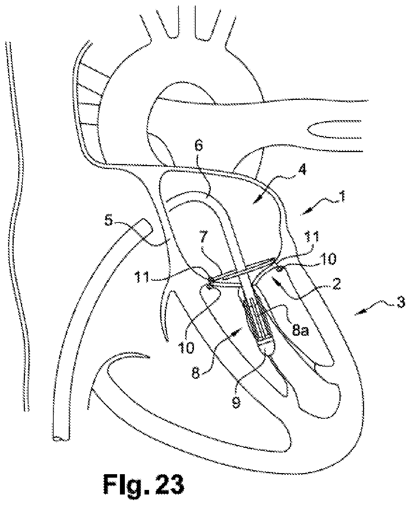

A partir de ce concept, deux formes de réalisation ont été réalisées, à savoir une première forme de réalisation illustrée aux figures 1 à 12 dans laquelle l'anneau de renfort (7) est fixé par le dessous de la valve mitrale, et une deuxième forme de réalisation illustrée aux figures 13 à 24 dans laquelle l'anneau de renfort (7) est fixé par le dessus de la valve mitrale.

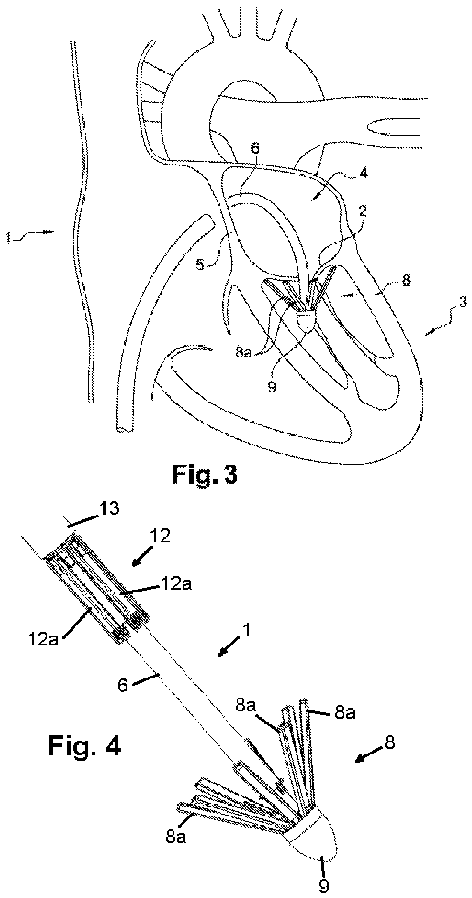

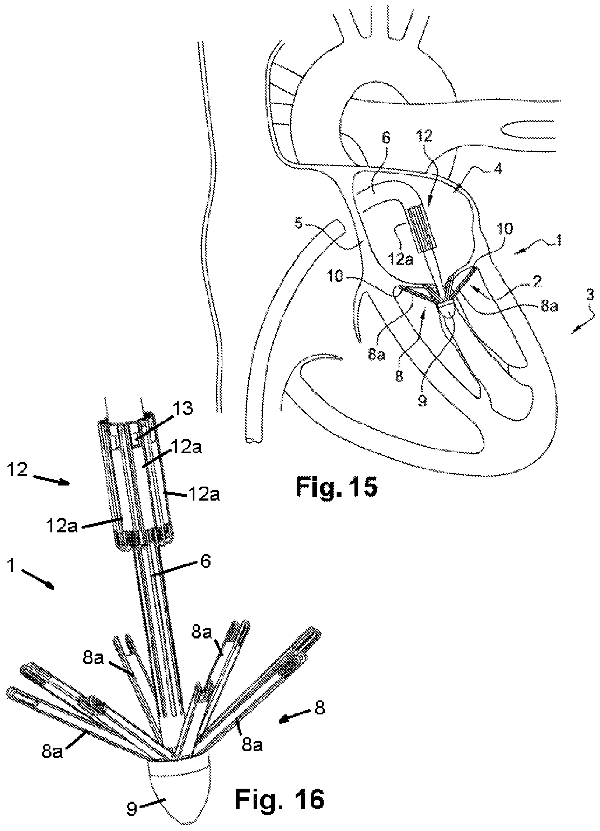

En référence aux figures 1 à 4, et 13 à 16, dans les deux formes de réalisation, l'ensemble comprend un organe d'appui (8) comportant une pluralité de bras (8a) reliés d'une manière pivotante à une ogive (9) disposée à l'extrémité de la tige (6). L'extrémité arrondie de l'ogive (9) est a-traumatique. Les bras (8a) sont articulés et montés pivotants pour passer, sous l'action des moyens de commande, d'une position repliée (Fig. 2 ou Fig. 14) le long de la tige (6) à une position déployée (Fig. 4 ou Fig. 16), à la manière d'un parapluie, écartée de la tige (6).

Ensuite, par traction sur la tige (6) et l'organe d'appui (8), les bras (8a) trouvent naturellement leur chemin à travers les cordages de la valve mitrale pour venir se placer juste en dessous de l'anneau mitral (2) de la valve mitrale, dans l'angle formé par les feuillets et la paroi cardiaque, pour y prendre appui de manière uniformément répartie le long de la périphérie de la valve mitrale, et y exercer une pression, voir Fig. 3 ou Fig. 15. Afin de faciliter le passage des bras (8a) de l'organe d'appui (8) au travers des cordages de la valve mitrale, chaque bras peut s'étendre selon une direction courbée ou être flexible pour pouvoir adopter une position courbée. Il est également envisageable de casser la longueur de chaque bras (8a) par un point d'articulation autorisant le pliage du bras (8a) autour dudit point d'articulation. Selon une autre forme de réalisation, non illustrée, l'extrémité de chaque bras (8a) peut être constituée de plusieurs branches, aptes à s'écarter les unes des autres après le passage des cordages de la valve mitrale pour former plusieurs points d'appui sous l'anneau mitral.

Dans la deuxième forme de réalisation de l'invention, chacune des extrémités libres des bras (8a) de l'organe d'appui (8) est conformée, par exemple en forme de boucle, pour chacune recevoir une pièce de textile (10) en feutre ou téflon, connue de l'homme du métier sous le terme anglais « pledget ». Les pièces de textile (10) sont destinées à être plaquées sous l'anneau mitral (2) et à être fixées par des sutures (11) en contre-appui de l'anneau de renfort (7), tel qu'il sera décrit plus bas.

Dans les deux formes de réalisation, les bras (8a) de l'organe d'appui (8) sont déployés manuellement, soit tous ensemble, soit de manière sélective et indépendamment les uns des autres pour passer plus facilement les cordages de l'appareil mitral et s'adapter à la géométrie de l'anneau mitral (2). Pour s'adapter davantage à la géométrie de l'anneau mitral (2), qui n'est pas forcément circulaire, les bras (8a) sont télescopiques pour présenter des longueurs différentes.

Selon une première variante, les bras (8a) peuvent être déployés par des moyens mécaniques (non représentés) que comprennent les moyens de commande. Par exemple, le vissage d'une molette peut permettre de déployer les bras (8a). Dans une autre variante, les bras (8a) sont auto-expansibles et sont insérés dans un fourreau (non représenté) lorsqu'ils sont dans la position repliée, et sont extraits dudit fourreau dans la position déployée. L'ogive (9) a-traumatique peut faire office de fourreau. Dans cette dernière configuration, les bras (8a) sont reliés de manière articulée à un support, tel qu'une douille, monté coulissant sur la tige (6) pour venir se loger dans l'ogive (9) en position repliée des bras (8a).

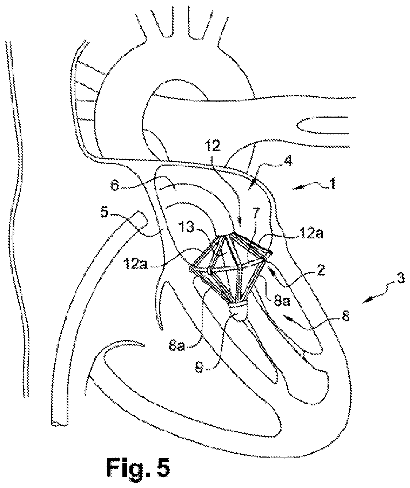

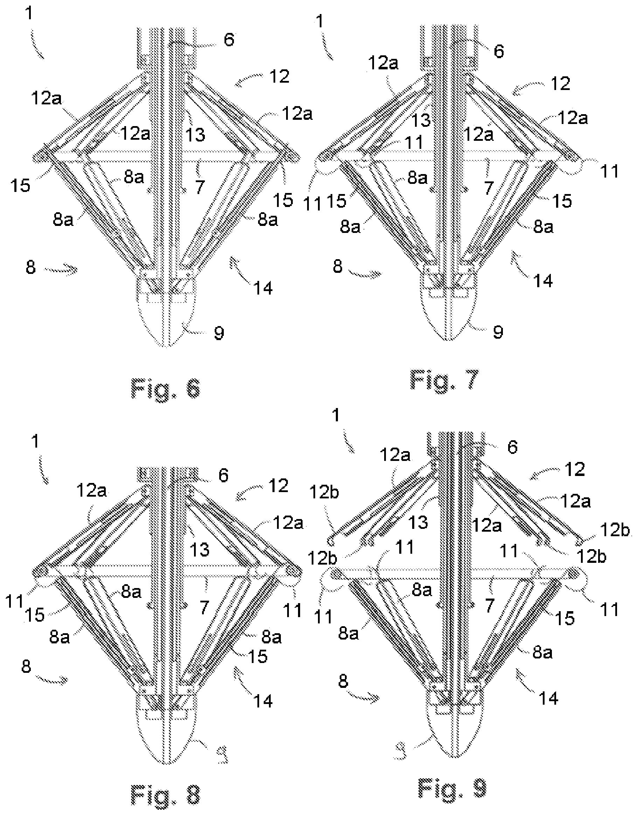

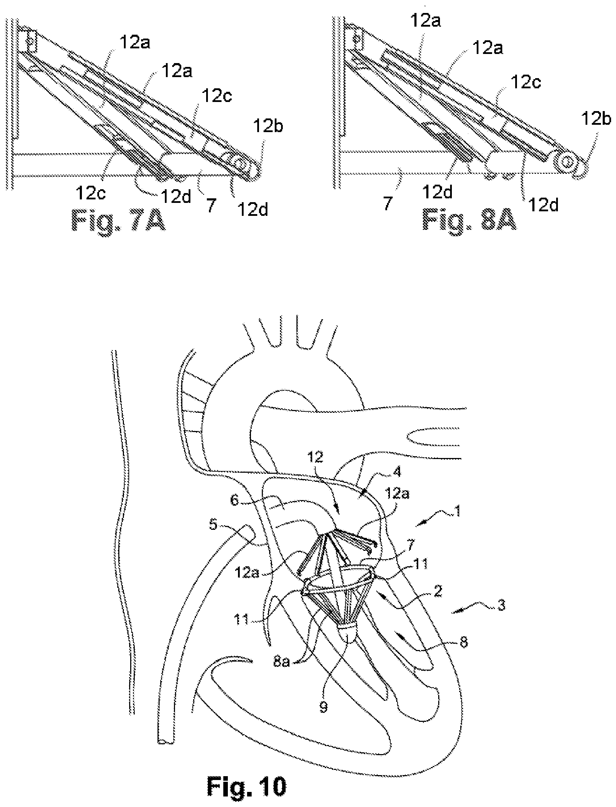

En référence aux figures 5 à 10, et 17 à 21, l'ensemble comprend un organe de contre-appui (12) comportant une pluralité de bras (12a), à l'extrémité libre desquels l'anneau de renfort (7) est maintenu. Plus précisément, chaque bras (12a) présente une extrémité libre formant une fourche (12b), voir figure 9, dont les extrémités sont conformées en crochets pour le maintien de l'anneau de renfort (7). En référence aux figures 7A et 8A, chaque bras (12a) comprend intérieurement un coulisseau (12c) prolongé par une spatule (12d). Le coulisseau (12c) est assujetti à des moyens (non représentés), tels qu'un câble par exemple, permettant de faire coulisser le coulisseau (12c) à l'intérieur du bras (12a) de sorte à faire passer la spatule (12d) d'une position de blocage de l'anneau de renfort (7) dans laquelle elle se trouve sous ce dernier (Fig. 7A), à une position retirée de relâchement de l'anneau de renfort (7) (Fig. 8A). La spatule (12d) se présente sous la forme de deux doigts parallèles définissant entre eux un passage pour une aiguille tel qu'il sera décrit plus bas.

Les bras (12a) de l'organe de contre-appui (12) sont reliés de manière pivotante à un support (13), tel qu'une douille, disposé coaxialement à la tige (6) et monté avec capacité de coulissement le long de celle-ci. Les bras (12a) de l'organe de contre-appui (12) sont articulés et montés pivotants de sorte à passer, sous l'action des moyens de commande, d'une position repliée le long de la tige (6) à une position déployée écartée de la tige (6) pour réaliser le contre-appui sur l'anneau mitral (2) et positionner l'anneau de renfort (7) en contre-pression et au droit des bras (8a) de l'organe d'appui (8). De la même manière que pour les bras (8a) de l'organe d'appui (8), les bras (12a) de l'organe de contre-appui (12) peuvent être déployés simultanément ou de manière sélective et indépendamment les uns des autres, être télescopiques, et être auto-expansibles et agencés dans un fourreau dans leur position repliée.

De ce qui précède, les bras (8a) de l'organe d'appui (8) et les bras (12a) de l'organe de contre-appui (12) s'ouvrent à la manière d'un parapluie et viennent en correspondance de part et d'autre de l'anneau mitral (2) pour former un appui/contre-appui. Cette caractéristique permet ensuite de percer l'anneau mitral (2) de façon simple, idéale et sans traction pour fixer l'anneau de renfort (7), tel qu'il sera décrit plus bas, par l'intermédiaire de moyens (14) d'extraction de sutures (11).

Comme évoqué plus haut, les deux formes de réalisation présentées diffèrent par la technique de fixation de l'anneau de renfort (7).

Selon la première forme de réalisation, et en référence aux figures 6 à 9, la fixation de l'anneau de renfort (7) est effectuée par le dessous de la valve mitrale et les sutures (11) sont extraites depuis les bras (8a) de l'organe d'appui (8) qui forment ainsi les moyens (14) d'extraction de sutures (11). A cet effet, chaque bras (8a) comprend intérieurement une aiguille (15) montée avec capacité de déplacement en translation pour déborder de l'extrémité libre des bras (8a), et libérer les sutures (11) pour fixer l'anneau de renfort (7). Les aiguilles (15) sont conformées pour permettre l'engagement, le guidage et le maintien des sutures (11) en débordement de l'aiguille (15).

Selon un premier exemple de mise en œuvre, les aiguilles (15) sont destinées à être extraites pour percer l'anneau mitral (2) et faire saillie de la face de dessus de l'anneau mitral (2), en débouchant à l'intérieur des doigts (12d) et des fourches (12b) des bras (12a) correspondants de l'organe de contre-appui (12). Ensuite, chaque suture (11) sous la forme d'un fil (11a) réalisé dans un matériau à mémoire de forme va automatiquement, après avoir été libéré de l'aiguille (15), être déformé pour créer une boucle d'ancrage autour de l'anneau de renfort (7) et dans l'épaisseur de l'anneau mitral (2) pour permettre la fixation dudit anneau de renfort (7) sur l'anneau mitral (2).

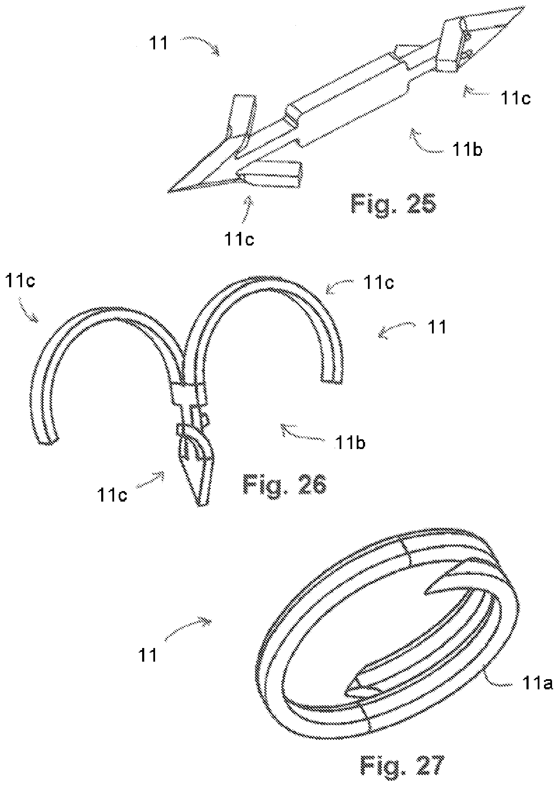

Selon un deuxième exemple de mise en œuvre, les aiguilles (15) ne percent pas l'anneau mitral (2) et délivrent des sutures (11) sous la forme d'agrafes (11b) qui sont conformées en pointe pour traverser d'elles même l'anneau mitral (2) et l'anneau de renfort (7), et qui comprennent des parties d'ancrage (11c), telles que des extrémités en arpon, pour interdire le retrait desdites agrafes (11b) et verrouiller la fixation de l'anneau de renfort (7) sur l'anneau mitral (2). Des exemples de réalisation des différentes agrafes (11b) et du fil (11a) sont illustrées figures 25 à 27.

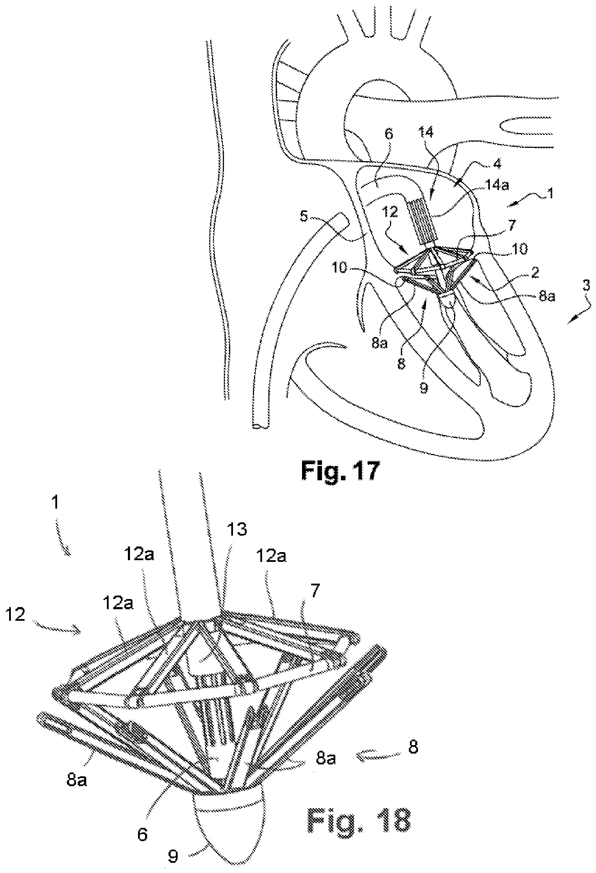

Selon la deuxième forme de réalisation, et en référence aux figures 17 à 21, la fixation de l'anneau de renfort (7) est effectuée par le dessus de l'anneau mitral (2). A cet effet, dans cette forme de réalisation les moyens (14) d'extraction de sutures (11) comprennent une pluralité de bras (14a) reliés de manière pivotante à un support (15), tel qu'une douille, disposé coaxialement à la tige (6) de sorte à passer, sous l'action des moyens de commande, d'une position repliée le long de la tige (6) à une position déployée écartée de la tige (6).

Lorsque les bras (14a) des moyens (14) d'extraction sont en position déployée, ils se trouvent en correspondance avec les bras (12a) de l'organe de contre-appui (12). Pour permettre la fixation de l'anneau de renfort (7), chaque bras (14a) comprend intérieurement, et de la même manière que pour la première forme de réalisation, une aiguille (15) pour délivrer une suture sous la forme d'un fil (11a) ou d'une agrafe (11b) prévue pour s'ancrer sur la périphérie de la valve mitrale et y fixer l'anneau de renfort (7). Le fonctionnement est identique et n'est pas décrit de nouveau. Dans cette forme de réalisation, les sutures (11) viennent fixer les pièces de textile (10) en renfort et en contre-appui de l'anneau de renfort (7), et de l'autre côté de l'anneau mitral (2). Les pièces de textiles (10) forment des renforts pour les sutures (11), de sorte que la tension de la suture (11) est répartie sur l'ensemble de la pièce de textile (10), et non directement sur l'anneau mitral (2). Les pièces de textile (10) assurent une protection contre les traumatismes dus à la tension de la suture (11).

De la même manière que pour les bras (8a) de l'organe d'appui (8) ou les bras (12a) de l'organe de contre-appui (12), les bras (14a) des moyens (14) d'extraction peuvent être déployés simultanément ou de manière sélective et indépendamment les uns des autres, être télescopiques, et être auto-expansibles et agencé dans un fourreau dans leur position repliée.

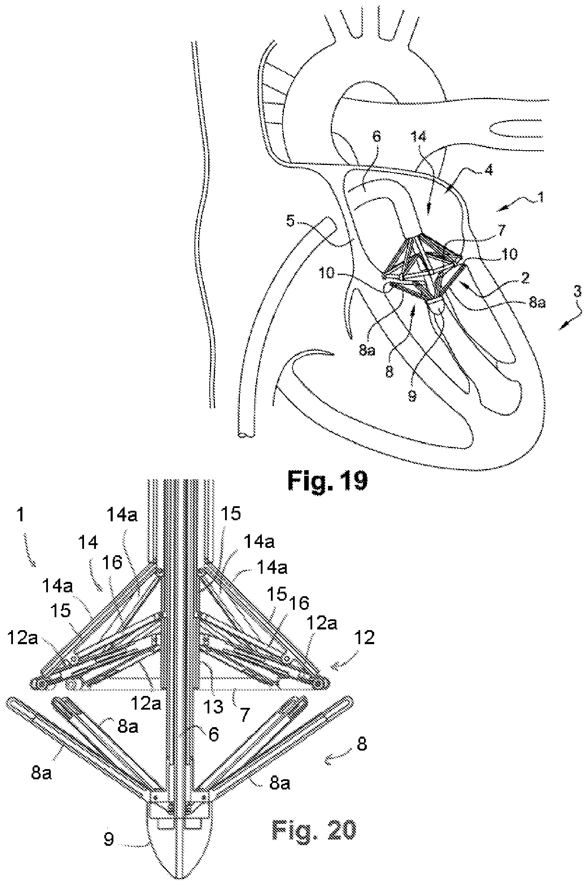

En référence à la figure 20, l'extrémité libre de chaque bras (14a) que comprennent les moyens (14) d'extraction est reliée à l'élément support (15) par une biellette (16) pour former un appui stable lors de l'opération de suture et de fixation de l'anneau de renfort (7).

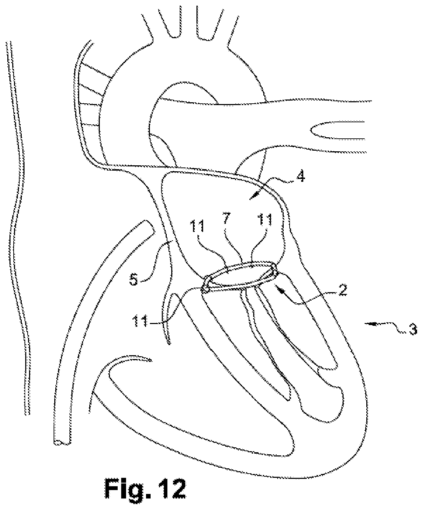

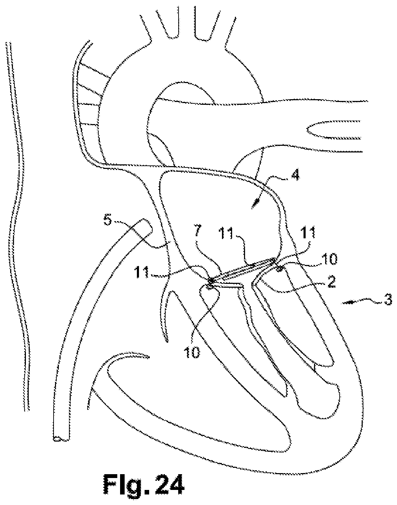

En référence aux figures 10 à 12, et 21 à 24, lorsque l'anneau de renfort (7) est enfin fixé, et que les spatules (12d) ont été retirées pour libérer l'anneau de renfort (7), les différents éléments de l'ensemble sont chacun à leur tour passé dans leur position repliée et sont un à un extrait du cœur (3). On aperçoit, figure 12 et 24, l'anneau de renfort (7) fixé sur l'anneau mitral (2).

L'anneau de renfort (7) constitue un implant prothétique apte à diminuer le calibre de l'anneau mitral (2) pour diminuer, voire supprimer les fuites mitrales. Suivant une autre caractéristique importante, l'anneau de renfort (7) est assujetti à un moyen (non représenté) apte à permettre de réduire sa circonférence après sa mise en place et fixation sur la périphérie de l'anneau mitral (2), comme indiqué. Par exemple, ces moyens sont constitués par un cordon de traction monté librement en translation et à libre coulissement dans l'âme centrale de l'anneau de renfort (7) pour permettre, sous un effet de traction, d'assurer le fronçage de l'anneau et, par conséquent, la diminution de son diamètre. Ces dispositions sont particulièrement importantes pour permettre après la fixation de l'anneau de renfort (7) dans les conditions indiquées, de parfaitement adapter le diamètre de l'implant en s'assurant qu'il n'y a plus aucune fuite.

Dans ce but, le chirurgien retire le dispositif (1), et seules dépassent de l'introducteur, les deux extrémités du fil (11a) qui constitue l'âme axiale de l'anneau. Sous contrôle radio, en exerçant une simple fraction sur le fil, on provoque de manière concomitante le serrage de l'anneau de la valve. Après avoir trouvé la bonne tension correspondant au bon diamètre, le fil peut être serti au moyen d'un nœud ou d'un clip, puis coupé.

Les caractéristiques du dispositif (1) selon l'invention apportent de nombreux avantages par rapport aux solutions existantes. Le fait de passer par la voie transfémorale permet de réduire la durée et le caractère invasif de l'opération. Avec une coupe échographique bidimensionnelle de la jonction auriculo ventriculaire, il est possible de visualiser l'enclavement souhaité du dispositif (1) sur la petite valve mitrale du côté ventriculaire. La mise en place est guidée par l'anatomie même des tissus et de la résistance que l'opérateur perçoit en position de butée de l'organe d'appui (8) et de l'organe de contre-appui (12)

A noter également que le dispositif (1) permet d'exercer deux forces d'appui et de contre-appui opposées pour effectuer la perforation des tissus et le passage des sutures (11) d'ancrage sans risque de fragiliser ledit tissu en supprimant tout risque de tâtonnement. Autrement dit, le tissu de l'anneau mitral (2) et les tissus adjacents sont perforés plus efficacement en une seule fois avec une tenue optimale des sutures (11).

A noter enfin que les bras (8a) de l'organe d'appui (8) et/ou les bras (12a) de l'organe de contre-appui (12) et/ou les bras (14a) des moyens (14) d'extraction comprennent chacun un marqueur radio-opaque et/ou échographique permettant au chirurgien d'effectuer un contrôle en 2D ou en 3D de leur position au fur et à mesure de l'opération.

## Revendications

1. Dispositif (1) pour réaliser ou préparer une annuloplastie par voie transfémorale de la valve mitrale d'un cœur, et destiné à être positionné dans un introducteur étanche disposé dans une veine fémorale pour pénétrer dans l'oreillette gauche du cœur en passant par sa paroi septale, comprenant un ensemble apte à coopérer avec une poignée assujettie à des moyens de commande pour l'actionnement de l'ensemble pour la mise en place et la fixation d'un anneau de renfort (7) sur l'anneau mitral, ledit ensemble est agencé à l'extrémité d'une tige (6) de manipulation et comprend :
- un organe d'appui (8) comprenant une pluralité de bras (8a) pouvant prendre appui sous l'anneau mitral de manière uniformément répartie le long de la périphérie de la valve mitrale ;
- un organe de contre-appui (12) comprenant une pluralité de bras (12a), à l'extrémité libre desquels est agencé l'anneau de renfort (7), les bras (12a) sont reliés de manière pivotante à un support (13) disposé coaxialement à la tige (6) de sorte à passer, sous l'action des moyens de commande, d'une position repliée le long de la tige (6) à une position déployée écartée de la tige (6) pour réaliser le contre-appui sur l'anneau mitral et positionner l'anneau de renfort (7) ;
***caractérisé* en ce que** la pluralité de bras (8a) de l'organe d'appui sont reliés de manière pivotante à l'extrémité de la tige (6) de sorte à passer, sous l'action des moyens de commande, d'une position repliée le long de la tige (6) à une position déployée écartée de la tige (6), et **en ce que** l'ensemble comprend des moyens (14) d'extraction de sutures (11) pour fixer l'anneau de renfort (7) sur l'anneau mitral.

2. Dispositif (1) selon la revendication 1, ***caractérisé* en ce que** les bras (8a) de l'organe d'appui (8) et/ou les bras (12a) de l'organe de contre-appui (12) sont reliés de manière pivotante au support (13) disposé coaxialement à la tige (6) de sorte à passer sous l'action des moyens de commande simultanément ou sélectivement et indépendamment les uns des autres de la position repliée à la position déployée.

3. Dispositif (1) selon la revendication 1, ***caractérisé* en ce que** les bras (8a) de l'organe d'appui (8) et/ou les bras (12a) de l'organe de contre-appui (12) sont disposés à l'intérieur d'un fourreau dans la position repliée, et sont poussés hors du fourreau et sont auto-expansibles dans la position déployée.

4. Dispositif (1) selon la revendication 1, ***caractérisé* en ce que** les bras (8a) de l'organe d'appui (8) et/ou les bras (12a) de l'organe de contre-appui (12) comprennent chacun un marqueur radio-opaque et/ou échographique.

5. Dispositif (1) selon la revendication 1, ***caractérisé* en ce que** les bras (8a) de l'organe d'appui (8) et/ou les bras (12a) de l'organe de contre-appui (12) sont télescopiques.

6. Dispositif (1) selon la revendication 1, ***caractérisé* en ce que** les moyens (14) d'extraction de sutures (11) comprennent une pluralité de bras (14a) reliés de manière pivotante à un support (15) disposé coaxialement à la tige (6) de sorte à passer, sous l'action des moyens de commande, d'une position repliée le long de la tige (6) à une position déployée écartée de la tige (6) en correspondance avec les bras (12a) de l'organe de contre-appui (12), chaque bras (14a) comprend intérieurement une suture (11) apte à être extraite pour s'ancrer sur la périphérie de la valve mitrale et y fixer l'anneau de renfort (7).

7. Dispositif (1) selon la revendication 1, ***caractérisé* en ce que** chaque bras (8a) de l'organe d'appui (8) est recourbé, articulé ou flexible pour faciliter son passage au travers des cordages de la valve mitrale.

8. Dispositif (1) selon la revendication 1, ***caractérisé* en ce que** l'extrémité de chaque bras (8a) peut être constituée de plusieurs branches, aptes à s'écarter les unes des autres après le passage des cordages de la valve mitrale pour former plusieurs points d'appui sous l'anneau mitral.

9. Dispositif (1) selon la revendication 1, ***caractérisé* en ce que** chaque bras (8a) de l'organe d'appui (8) comprend intérieurement une suture (11) apte à être extraite pour traverser l'anneau mitral et fixer l'anneau de renfort (7).

10. Dispositif (1) selon la revendication 6, ***caractérisé* en ce que** l'extrémité libre de chaque bras (8a) de l'organe d'appui (8) comprend une pièce de textile (10), en feutre ou en téflon, destinée à être plaquée sous l'anneau mitral et à être fixée par les sutures (11) en renfort et en contre-appui de l'anneau de renfort (7).

## Patentansprüche

1. Vorrichtung (1) zur Ausführung oder Vorbereitung einer Annuloplastie auf transfemoralem Weg der Mitralklappe eines Herzens, und dazu bestimmt, in eine dichte Einführungsvorrichtung eingesetzt zu werden, die in einer Femoralvene positioniert ist um in den linken Vorhof des Herzens einzudringen, durch seine Septalwand hindurch, mit einer Baugruppe, die mit einem Griff zusammenarbeiten kann, der über Steuermittel bedient wird, zur Betätigung der Baugruppe zum Einsetzen und zur Befestigung eines Verstärkungsrings (7) auf dem Mitralring, diese Baugruppe ist am Ende einer Manipulatorstange (6) angeordnet und umfasst:
- ein Stützelement (8) mit mehreren Armen (8a), die sich unter dem Mitralring gleichmäßig über die Peripherie der Mitralklappe verteilt abstützen können;
- ein Widerlager (12), mit mehreren Armen (12a), an deren freien Ende der Verstärkungsring (7) vorgesehen ist, die Arme (12a), sind kippbar mit einem Halter (13) verbunden, der koaxial zur Stange (6) angeordnet ist, so dass sie unter der Wirkung der Steuermittel aus einer entlang der Stange (6) eingeklappten Position in eine zur Stange (6) abgewinkelte Position geklappt werden können, um das Widerlager zum Mitralring zu bilden und den Verstärkungsring (7) zu positionieren, ***dadurch gekennzeichnet, dass*** die mehreren Arme (8a) des Stützelementes kippbar mit dem Endstück der Stange (6) verbunden sind, so dass sie unter der Wirkung der Steuermittel aus einer entlang der Stange (6) eingeklappten Position in eine zur Stange (6) abgewinkelte Position geklappt werden können, und ***dadurch dass*** die Baugruppe Mittel (14) zur Extraktion von Nahtmaterial (11) enthält, um den Verstärkungsring (7) am Mitralring zu befestigen.

2. Vorrichtung (1) nach Anspruch 1, ***dadurch gekennzeichnet, dass*** die Arme (8a) des Stützelementes (8) und/ oder die Arme (12a) des Widerlagers (12) kippbar mit dem Halter (13) verbunden sind, der koaxial zur Stange (6) angeordnet ist, so dass sie unter der Wirkung der Steuermittel gleichzeitig oder selektiv und unabhängig voneinander aus der eingeklappten Position in die abgewinkelte Position geklappt werden können.

3. Vorrichtung (1) nach Anspruch 1, ***dadurch gekennzeichnet, dass*** die Arme (8a) des Stützelementes (8) und/ oder die Arme (12a) des Widerlagers in der eingeklappten Position innerhalb einer Hülse untergebracht werden, aus der sie hinausgestoßen werden können und sich von selbst in die abgewinkelte Position ausklappen.

4. Vorrichtung (1) nach Anspruch 1, ***dadurch gekennzeichnet, dass*** die Arme (8a) des Stützelementes (8) und/ oder die Arme (12a) des Widerlagers (12) jeweils einen für Strahlen undurchlässigen und/ oder Ultraschall- Marker enthalten.

5. Vorrichtung (1) nach Anspruch 1, **dadurch gekennzeichnet, dass** die Arme (8a) des Stützelementes (8) und/ oder die Arme (12a) des Widerlagers (12) Teleskoparme sind.

6. Vorrichtung (1) nach Anspruch 1, ***dadurch gekennzeichnet, dass*** die Mittel (14) zur Extraktion des Nahtmaterials (11) mehrere Arme (14a) enthalten, die kippbar mit einem Halter (15) verbunden sind, der koaxial zur Stange (6) angeordnet ist, so dass sie unter der Wirkung des Steuermittels aus einer entlang der Stange (6) eingeklappten Position in eine zur Stange (6) abgewinkelte Position geklappt werden können, entsprechend den Armen (12a) des Widerlagers (12), jeder Arm (14a) enthält in seinem Inneren Nahtmaterial (11), das extrahiert werden kann, um an der Peripherie der Mitralklappe verankert zu werden und dort den Verstärkungsring (7) zu befestigen.

7. Vorrichtung (1) nach Anspruch 1, ***dadurch gekennzeichnet, dass*** jeder Arm (8a) des Stützelementes (8) gekrümmt, abgewinkelt, mit Gelenken versehen oder flexibel ist, um das Hindurchführen durch die Sehnenfäden der Mitralklappe zu erleichtern.

8. Vorrichtung (1) nach Anspruch 1, ***dadurch gekennzeichnet, dass*** das Endstück jedes Arms (8a) aus mehreren Ästen bestehen kann, die sich voneinander abspreizen können, nachdem sie durch die Sehnenfäden der Mitralklappe hindurchgeführt wurden, um mehrere Abstützpunkte auf dem Mitralring zu bilden.

9. Vorrichtung (1) nach Anspruch 1, ***dadurch gekennzeichnet, dass*** jeder Arm (8a) des Stützelementes (8) in seinem Inneren Nahtmaterial (11) enthält, das extrahiert werden kann, um durch den Mitralring hindurchzuführen und den Verstärkungsring (7) zu befestigen.

10. Vorrichtung (1) nach Anspruch 6, **dadurch gekennzeichnet, dass** das freie Endstück jedes Arms (8a) des Stützelementes (8) ein Stück Textil (10) aus Filz oder Teflon enthält, das gegen den Mitralring gepresst werden und mit dem Nahtmaterial (11) als Verstärkung und als Widerlager des Verstärkungsrings (7) befestigt werden soll.

## Claims

1. Device (1) for performing or preparing a transfemoral annuloplasty of the mitral valve of a heart, and intended to be positioned in a sealed introducer placed in a femoral vein to penetrate the left atrium of the heart through its septal wall, comprising an assembly for cooperating with a handle under the control of a control means for actuating the assembly for placing and attaching a reinforcement ring (7) on the mitral annulus, with said assembly arranged at the end of a handling rod (6) and comprising:
- a bearing member (8) comprising a plurality of arms (8a) able to provide support under the mitral annulus (2) in a manner uniformly distributed along the periphery of the mitral valve;
- a counter-bearing member (12) comprising a plurality of arms (12a), at the free end of which arms the reinforcement ring (7) is arranged, with the arms (12a) connected pivotably to a support (13) disposed coaxially with respect to the rod (6) in such a way as to change, under the action of the control means, from a position folded along the rod (6) to a deployed position spaced away from the rod (6) in order to realize the countersupport on the mitral annulus and position the reinforcement ring (7);
**characterized in that** the plurality of arms (8a) are connected pivotably to the end of the rod (6) so as to change, under the action of the control means, from a position folded along the rod (6) to a deployed position spaced away from the rod (6), and **in that** the assembly comprises means (14) for removing sutures (11) to attach the reinforcement ring (7) to the mitral annulus.

2. Device (1) according to claim 1, **characterized in that** the arms (8a) of the bearing member (8) and/or the arms (12a) of the counter-bearing member (12) are connected pivotably to a support (13) disposed coaxially with respect to the rod (6) in such a way as to change, under the action of the control means, simultaneously or selectively and independently from each other, from the folded position to the deployed position.

3. Device (1) according to claim 1, **characterized in that** the arms (8a) of the bearing member (8) and/or the arms (12a) of the counter-bearing member (12) are arranged inside a sheath in the folded position, and are pushed out of the sheath and are self-expanding in the deployed position.

4. Device (1) according to claim 1, **characterized in that** the arms (8a) of the bearing member (8) and/or the arms (12a) of the counter-bearing member (12) each include a radiopaque and/or ultrasound marker.

5. Device (1) according to claim 1, **characterized in that** the arms (8a) of the bearing member (8) and/or the arms (12a) of the counter-bearing member (12) are telescopic.

6. Device (1) according to claim 1, **characterized in that** the means (14) to allow the extraction of sutures (11) comprise a plurality of arms (14a) connected pivotably to a support (15) disposed coaxially to the rod (6) so as to change, under the action of the control means, from a folded position along the rod (6) to a deployed position spaced away from the rod (6) that aligns with the arms (12a) of the counter-bearing member (12), each arm (14a) internally comprising an extractable suture (11) to anchor on the periphery of the mitral valve and to fix the reinforcement ring thereto (7).

7. Device (1) according to claim 1, **characterized in that** each arm (8a) of the bearing member (8) is curved, articulated, or flexible to facilitate its passage through the tendinous cords of the mitral valve.

8. Device (1) according to claim 1, **characterized in that** the end of each arm (8a) may be composed of several branches, which can move away from each other after passing through the mitral valve cords to form several support points under the mitral annulus.

9. Device (1) according to claim 1, **characterized in that** each arm (8a) of the bearing member (8) internally comprises an extractable suture (11) to go through the mitral annulus and attach the reinforcement ring (7).

10. Device (1) according to claim 6, **characterized in that** the free end of each arm (8a) of the bearing member (8) comprises a piece of felt or Teflon fabric (10) intended to be laid under the mitral valve and fixed by the sutures (11) to support and counter-bear against the reinforcement ring (7).
